Europäisches Patentamt

European Patent Office

Office européen des brevets

(11) Publication number: **0 318 446 B1**

# EUROPEAN PATENT SPECIFICATION

(45) Date of publication of patent specification: **14.10.92**    (51) Int. Cl.5: **A61K 37/18**

(21) Application number: **88850373.7**

(22) Date of filing: **28.10.88**

Consolidated with 88909629.3/0398879
(European application No./publication No.) by
decision dated 25.03.91.

(54) **Amino acid composition for parenteral nutritional support.**

(30) Priority: **29.10.87 SE 8704217**

(43) Date of publication of application:
**31.05.89 Bulletin 89/22**

(45) Publication of the grant of the patent:
**14.10.92 Bulletin 92/42**

(84) Designated Contracting States:
**AT BE CH DE ES FR GB GR IT LI LU NL SE**

(56) References cited:
**WO-A-87/03806**

**DIALOG 05710808, Medline 86011808; C.R.
KAPADIA: "Maintenance of skeletal muscle
intracellular glutamine during standard sur-
gical trauma"**

**The Lancet, vol. 335, p. 701-703 (1990)**

(73) Proprietor: **AB ERIK VINNARS
Öregrundsgatan 24
S-115 28 Stockholm(SE)**

(72) Inventor: **Vinnars, Erik
Bisslinge Gard
S-191 77 Sollentuna(SE)**

(74) Representative: **Rostovanyi, Peter et al
AWAPATENT AB Box 5117
S-200 71 Malmö(SE)**

EP 0 318 446 B1

## Description

The present invention relates to a composition for therapeutic, especially postoperative and post-traumatic, parenteral nutritional support treatment, which composition, in addition to conventional amino acid components, also contains alpha-ketoglutarate, and optionally L-asparagine and acetoacetate.

In states of illness, surgical operations and injuries, profound changes are induced in the energy and protein metabolism of the human body. This means, for example, loss of active cellular mass, leading to muscular fatigue, pronounced apathy and loss of appetite, and a period of convalescence involving general weariness which, for instance after a biliary tract operation, may last 5-6 weeks before the patient has regained his normal function. The cellular mass which is broken down very rapidly in different states of illness will need a time for reestablishment which is about four times as long as the time of breakdown for the same mass.

In severe states of illness and injuries, and in postoperative states, parenteral nutritional support is generally applied. In the past, preparations for intraveneous nutritional support generally contained an aqueous solution of a high caloric content carbohydrate, such as glucose, and electrolytes. In prolonged states of illness or in injuries and surgical operations, the nitrogen balance of the body must however be considered, i.e. the ratio of nitrogen loss to nitrogen intake. In the case of negative nitrogen balance, the parenteral nutritional support can be supplemented with amino acid supply to improve the nitrogen balance. Different amino acid compositions for parenteral supply are previously known, see e.g. SE Patent Application 8203965 and DE-A 25 30 246 concerning amino acid nutrient compositions in renal failure, WO 82/00411 concerning a nutrient composition containing branched-chain amino acids, WO 83/03969 concerning an aqueous nutrient solution consisting of L-amino acids and WO 87/03806 concerning a parenteral nutrition aqueous solution containing glutamine together with other organic nitrogen containing compounds, such as alpha-ketoglutarate in a very small amount. In particular, the role of glutamine which may include an increase in organ functional capacity is emphasized.

From a survey made of the free amino acid pattern in the muscles, it has been found that skeletal muscle, which is the major body tissue in respect of weight, has a free amino acid pool, 62% of which consists of glutamine, see Bergström et al.: Intracellular free amino acid concentration in human muscle tissue, J. of Appl. Physiol., Vol 36, No 6, 1974. In states of illness, injuries or surgical operations, this content decreases by at least 50% and, in states of blood poisoning, even more, see Vinnars et al: Influence of the postoperative state on the intracellular free amino acids in human muscle tissue. Annals of Surg., Vol 182, 6:665-671, 1975.

It has been found that this glutamine reduction cannot be affected by enteral or parenteral nutritional support according to the methods hitherto available, see Vinnars et al.: Metabolic effects of four intravenous nutritional regiments in patients undergoing elective surgery. II. Muscle amino acids and energy rich phosphates. Clin. Nutr. 2:3-11, 1983. There probably is a correlation between the inability immediately postoperatively to make a negative nitrogen balance positive, the inability to normalise the exhausted intracellular glutamine pool, and the reduced muscular strength. This reduction probably depends on a reduced protein synthesis capacity posttraumatically in skeletal muscle, see Wernerman et al: Protein synthesis after trauma as studied by muscle ribosome profiles. Proceedings in the 7th ESPEN Congress. Ed. Dietze et al, Karger, Basel.

The addition to the nutritional support of a dipeptide of the type ornithine-alpha-ketoglutarate to a commercial amino acid solution has been found to improve to some extent, whereas not to normalise the intracellular glutamine pool, see Leander et al: Nitrogen sparing effect of Ornicetil in the immediate postoperative state. Clin. Nutr. 4:43-51, 1985. This preparation is however very expensive, and it has not been possible so far to evaluate whether its use in parenteral nutrition confers a clinical advantage.

Postoperatively, the patient often exhibits loss of appetite, making it difficult to supply nutrition, although there are possibilities, by tube-feeding, of supplying different kinds of nutrient solutions. Since most patients do not tolerate this way of feeding, it becomes necessary to resort to intravenous feeding. The nutrition substrates available for energy metabolism are various sugar solutions and fatty emulsions, which today seem appropriate. However, the amino acid solutions available are inadequate, both because it is not possible to add tyrosine in sufficient amounts since this is a relatively insoluble amino acid, and because certain important amide derivatives of amino acids (glutamine and asparagine) cannot be included. This is due to difficulties in heat-sterilising solutions of such amides, and also to the fact that the amides are unstable when stored. Another problem is that these compounds are relatively sparingly soluble and therefore require large amounts of water when being prepared.

After elective surgery, for instance biliary tract operations, it has been found that the negative nitrogen balance primarily depends on reduced protein synthesis which is assessed by determining the ribosome activity in skeletal muscle, see Wernerman et al: Protein synthesis in skeletal muscle after abdominal surgery: The effect of total parenteral nutrition Journal of Parenteral Enteral Nutrition (1986) vol. 10, p.578-582. An increased protein breakdown occurs only in very severe traumas and primarily in septic states. This reduced protein synthesis capacity cannot be affected by conventional intravenous or enteral nutritional support.

It has now been demonstrated for the first time that the addition of alpha-ketoglutarate, and optionally L-asparagine, to a parenteral nutrition program can prevent such reduction of the protein synthesis capacity and, hence, also improve the nitrogen balance and even make it positive. The abnormal animo acid pattern intracellularly in skeletal muscle, and especially the 50% reduction of the glutamine pool involved, can then be partially prevented.

Preliminary tests on patients subjected to a biliary tract operation have shown that an addition of alpha-ketoglutarate, and optionally an addition of L-asparagine or its keto derivative, acetoacetate, to a conventional parenteral nutritional support program improves the nitrogen balance of the patients and hence also their recovery to a great extent. Besides, the pathological amino acid changes which normally occur after injury or surgical operation are normalised and, also, the reduction of the ribosome acitivity is prevented. Obviously, this is the first time it has been possible more specifically to act on the negative effects of injury or surgical operation on the protein metabolism.

It has thus been found that said vital amide derivatives can be brought into a form suitable for administration by sterile filtration of an aqueous solution, followed by rapid cooling and cold storage limited to a few months. One alternative is freeze-drying the sterile-filtered solution, yielding a sterile powder. Immediately before administration, this powder can be added to a conventional amino acid mixture. Also other forms of powder sterilisation, not relying on heat, are conceivable. The possibility of using the Na salt of the compounds in order to increase the solubility has also been considered.

The invention thus relates to a composition for therapeutic, especially postoperative and posttraumatic, parenteral nutritional support treatment, which composition is based on a conventional amino acid mixture, the composition comprising alpha-ketoglutarate, and optionally L-asparagine and/or acetoacetate, the components of the composition, expressed in g dry component/l aqueous solution, being:

| glycine | 1-12 |
|---|---|
| aspartate | 1-10 |
| glutamate | 2-12 |
| alanine | 2-20 |
| arginine | 2-14 |
| cysteine/cystine | 0.4-2.0 |
| histidine | 2-8 |
| isoleucine | 2-8 |
| leucine | 2-8 |
| lysine | 2-12 |
| methionine | 1-6 |
| phenylalanine | 4-10 |
| proline | 4-10 |
| serine | 2-10 |
| threonine | 2-8 |
| tryptophan | 1-3 |
| tyrosine | 0.2-1 |
| valine | 2-8, |

the composition being characterised in that it also contains 5-25 g/l alpha-ketoglutarate, and optionally 0.5-10 g/l L-asparagine, 0.5-10 g/l acetoacetate and 5-30 g/l L-glutamine or salts or esters thereof.

A preferred amount of L-glutamine in the composition of the present invention is 10-30 g/l and an especially preferred amount is 15-25 g/l, specifically 20 g/l. A preferred amount of alpha-ketoglutarate in the composition of the present invention is 10-25 g/l, specifically 16.5 g/l.

In therapeutic tests, preferred compositions have included the following suitable components (expressed in g dry component/l aqueous solution):

| Example | 2 | 3 | 4 | 5 |
|---|---|---|---|---|
| glycine | 5.9 | 5.9 | 5.9 | 5.9 |
| aspartate | 4.8 | 4.8 | 4.8 | 4.8 |
| glutamate | 6.8 | 6.8 | 6.8 | 6.8 |
| alanine | 12 | 12 | 12 | 12 |
| arginine | 8.4 | 8.4 | 8.4 | 8.4 |
| cysteine/cystine | 0.42 | 0.42 | 0.42 | 0.42 |
| histidine | 5.1 | 5.1 | 5.1 | 5.1 |
| isoleucine | 4.2 | 4.2 | 4.2 | 4.2 |
| leucine | 5.9 | 5.9 | 5.9 | 5.9 |
| lysine | 6.8 | 6.8 | 6.8 | 6.8 |
| methionine | 4.2 | 4.2 | 4.2 | 4.2 |
| phenylalanine | 5.9 | 5.9 | 5.9 | 5.9 |
| proline | 8.0 | 8.0 | 8.0 | 8.0 |
| serine | 6.0 | 6.0 | 6.0 | 6.0 |
| threonine | 4.2 | 4.2 | 4.2 | 4.2 |
| tryptophan | 1.4 | 1.4 | 1.4 | 1.4 |
| tyrosine | 0.4 | 0.4 | 0.4 | 0.4 |
| valine | 5.5 | 5.5 | 5.5 | 5.5 |
| L-glutamine | - | 10 | 10 | 10 |
| alpha-ketoglutarate | 16.5 | 10 | 10 | 10 |
| asparagine | - | - | 2 | 4 |
| acetoacetate | - | - | 2 | - |

Alpha-ketoglutarate must be added to the composition in the form of its sodium salt or its esters, since it is otherwise extremely sparingly soluble. The glutamine can also be added in the form of the sodium salt thereof, thus improving its solubility.

The present application also relates to a method for preparing the composition as previously described and characterized in that alpha-ketoglutarate and optionally L-asparagine and/or acetoacetate or salts or esters thereof, dissolved in water and sterile-filtered, are added in cold-stored and freeze-dried form or other sterile powder form to a commercial amino acid nutrient solution immediately before the therapeutic treatment.

Implementation of the invention

Commercial forms of L-glutamine, alpha-ketoglutarate and optionally L-asparagine and/or acetoacetate or salts or esters thereof are dissolved in sterile pyrogen-free water at 30-50°C. The solution is sterile-filtered and rapidly cooled and may thereafter be stored for a few months in a solution in a cooled state or for an even longer time in the frozen state, or stored after freeze-drying for several years in sterile powder form, until it should be used together with an amino acid solution of conventional commercial type, for instance of the Vamin® type (amino acid nutrient composition from KabiVitrum AB). Carbohydrates and fatty substances can also be added to the infusion solution. Alpha-ketogutarate must be added in the form of its sodium salt or its esters, which is also possible, but not necessary, in the case of L-glutamine.

A newly prepared composition as above, either in large bags or in separate vials for each substrate, is then administered to patients exhibiting disordered nitrogen balance, resulting either from a surgical operation or from an injury or illness, the administration being conducted during a period of from 2-4 days to several weeks or until the patient can start eating ordinary food, with a dosage of 120-170 kJ/kg body weight/day, including 0.1-0.2 g amino acid nitrogen/kg body weight/day.

Example

In a study of patients who had been subjected to a biliary tract operation, the patients were divided into a test group of 8 persons and a control group of 9 persons. All patients were subjected to a parenteral nutritional treatment program (TPN) with an intake of intravenous liquids of 35 ml/kg body weight/day and an energy intake corresponding to 135 kJ/kg body weight/day in the form of equal parts of fat (Intralipid 20%, KabiVitrum) and carbohydrate (Glukos 10%, Pharmacia Infusion). The control group was given 0.2 g amino acid nitrogen/kg body weight/day in the form of Vamin® (KabiVitrum). The test group was also given

4

0.136 g alpha-ketoglutarate ($\alpha$-KG)/kg body weight/day. Thus, both groups were given isonitrogenous and isocaloric amounts of amino acid and energy. Electrolytes, tracer metals and vitamins were administered to both groups.

The daily nitrogen balance in means ± SEM was as follows:

```
α-KG        -0.54+0.53   -1.65+1.86   -0.15+1.07   -2.33+1.30

Control     -3.64+0.64   -2.83+0.86   -3.16+0.70   -9.57+1.30

Signifi-                              *(p<0.05)    *(p<0.05)
cance       **(p<0.01)
```

The glutamine concentration (in mmole/kg wet weight) in skeletal muscle was affected in the following manner.

```
            Preoperatively   3rd postoperative           Δ
                                  day

α-KG        15.42+0.79       11.75+0.87            -3.67+0.49

Control     14.58+1.39        8.71+0.83  p<0.05    -5.87+0.91
```

The present amino acid nutrient compositions thus have a very favourable effect on postoperative and posttraumatic states since they provide an improved nitrogen balance and unaltered protein synthesis capacity and, hence, promote a considerably quicker and improved recovery of patients than is the case of previously known amino acid nutrient compositions.

**Claims**

**Claims for the following Contracting States : AT, BE, CH, DE, FR, GB, IT, LI, LU, NL, SE**

1. Composition for therapeutic, especially postoperative and posttraumatic, parenteral nutritional support treatment, which composition is based on a conventional amino acid mixture comprising the following components, expressed in g dry component/l aqueous solution:

| | |
|---|---|
| glycine | 1-12 |
| aspartate | 1-10 |
| glutamate | 2-12 |
| alanine | 2-20 |
| arginine | 2-14 |
| cysteine/cystine | 0.4-2.0 |
| histidine | 2-8 |
| isoleucine | 2-8 |
| leucine | 2-8 |
| lysine | 2-12 |
| methionine | 1-6 |
| phenylalanine | 4-10 |
| proline | 4-10 |
| serine | 2-10 |
| threonine | 2-8 |
| tryptophan | 1-3 |
| tyrosine | 0.2-1 |
| valine | 2-8 |

**characterised** in that the composition also contains 5-25 g/l alpha-ketoglutarate, and optionally 0.5-10 g/l L-asparagine, 0.5-10 g/l acetoacetate, 5-30 g/l L-glutamine, or salts or esters thereof.

**2.** Composition as claimed in claim 1, **characterised** in that it contains 5-25, preferably 10-25 and most preferably 16.5 g/l alpha-ketoglutarate, and optionally 0.5-10 g/l L-asparagine and optionally 0.5-10 g/l acetoacetate or salts or esters thereof.

**3.** Composition as claimed in claim 1 or 2, **characterised** in that alpha-ketoglutarate is supplied in the form of the sodium salt therof.

**4.** Method for preparing the composition as claimed in any one of the preceding claims, **characterised** in that alpha-ketoglutarate and optionally L-asparagine and optionally acetoacetate are dissolved in water and sterile-filtered and added, in cold-stored or freeze-dried form or other sterile powder form, to the conventional amino acid mixture immediately before the therapeutic treatment.

**5.** The use of the composition as claimed in any one of claims 1-3 for preparing a pharmaceutical preparation for therapeutic, especially postoperative and posttraumatic, parenteral nutritional support treatment.

**Claims for the following Contracting States : ES, GR**

**1.** Method for preparing a composition for therapeutic, especially postoperative and posttraumatic, parenteral nutritional support treatment, which composition is based on a conventional amino acid mixture comprising the following components, expressed in g dry component/l aqueous solution:

| | |
|---|---|
| glycine | 1-12 |
| aspartate | 1-10 |
| glutamate | 2-12 |
| alanine | 2-20 |
| arginine | 2-14 |
| cysteine/cystine | 0.4-2.0 |
| histidine | 2-8 |
| isoleucine | 2-8 |
| leucine | 2-8 |
| lysine | 2-12 |
| methionine | 1-6 |
| phenylalanine | 4-10 |
| proline | 4-10 |
| serine | 2-10 |
| threonine | 2-8 |
| tryptophan | 1-3 |
| tyrosine | 0.2-1 |
| valine | 2-8 |

**characterised** in that 5-25 g/l alpha-ketoglutarate, and optionally 0.5-10 g/l L-asparagine, 0.5-10 g/l acetoacetate, 5-30 g/l L-glutamine, or salts or esters thereof are dissolved in water and sterile-filtered and added, in cold-stored or freeze-dried form or other sterile powder form, to the conventional amino acid mixture immediately before the therapeutic treatment.

**2.** Method as claimed in claim 1, **characterised** in that 5-25, preferably 10-25 and most preferably 16.5 g/l alpha-ketoglutarate, and optionally 0.5-10 g/l L-asparagine and 0.5-10 g/l acetoacetate or salts or esters thereof are dissolved in water and sterile-filtered and added, in cold-stored or freeze-dried form or other sterile powder form, to the conventional amino acid mixture immediately before the therapeutic treatment.

**3.** Method as claimed in claim 1 or 2, **characterised** in that alpha-ketoglutarate is supplied in the form of the sodium salt therof.

**4.** The use of a composition for therapeutic, especially postoperative and posttraumatic, parenteral nutritional support treatment, which composition is based on a conventional amino acid mixture

EP 0 318 446 B1

comprising the following components, expressed in g dry component/l aqueous solution:

| | |
|---|---|
| glycine | 1-12 |
| aspartate | 1-10 |
| glutamate | 2-12 |
| alanine | 2-20 |
| arginine | 2-14 |
| cysteine/cystine | 0.4-2.0 |
| histidine | 2-8 |
| isoleucine | 2-8 |
| leucine | 2-8 |
| lysine | 2-12 |
| methionine | 1-6 |
| phenylalanine | 4-10 |
| proline | 4-10 |
| serine | 2-10 |
| threonine | 2-8 |
| tryptophan | 1-3 |
| tyrosine | 0.2-1 |
| valine | 2-8 |

wherein the composition also contains 5-25 g/l alpha-ketoglutarate, and optionally 0.5-10 g/l L-asparagine, 0.5-10 g/l acetoacetate, 5-30 g/l L-glutamine, or salts or esters thereof.

5. The use as claimed in claim 4, wherein the composition contains 5-25, preferably 10-25 and most preferably 16.5 g/l alpha-ketoglutarate, and optionally 0.5-10 g/l L-asparagine and 0.5-10 g/l acetoacetate or salts or esters thereof.

6. The use as claimed in claim 4 or 5, wherein alpha-ketoglutarate is supplied in the form of the sodium salt thereof.

**Patentansprüche**
**Patentansprüche für folgende Vertragsstaaten : AT, BE, CH, DE, FR, GB, IT, LI, LU, NL, SE**

1. Zusammensetzung zur therapeutischen, insbesondere postoperativen und posttraumatischen, parenteralen Ernährungsbehandlung, auf Basis eines herkömmlichen Aminosäuregemisches, das - ausgedrückt in g trockener Bestandteil/l wässrige Lösung - wie folgt zusammengesetzt ist:

7

| Glycin | 1-12 |
|---|---|
| Aspartat | 1-10 |
| Glutamat | 2-12 |
| Alanin | 2-20 |
| Arginin | 2-14 |
| Cystein/Cystin | 0,4-2,0 |
| Histidin | 2-8 |
| Isoleucin | 2-8 |
| Leucin | 2-8 |
| Lysin | 2-12 |
| Methionin | 1-6 |
| Phenylalanin | 4-10 |
| Prolin | 4-10 |
| Serin | 2-10 |
| Threonin | 2-8 |
| Tryptophan | 1-3 |
| Tyrosin | 0,2-1 |
| Valin | 2-8 |

dadurch **gekennzeichnet,** dass die Zusammensetzung auch 5-25 g/l Alpha-Ketoglutarat und wahlweise 0,5-10 g/l L-Asparagin, 0,5-10 g/l Acetacetat, 5-30 g/l L-Glutamin oder deren Salze oder Ester enthält.

2. Zusammensetzung nach Anspruch 1, dadurch **gekennzeichnet,** dass sie 5-25, vorzugsweise 10-25 und möglichst 16,5 g/l Alpha-Ketoglutarat und wahlweise 0,5-10 g/l L-Asparagin und wahlweise 0,5-10 g/l Acetacetat oder deren Salze oder Ester enthält.

3. Zusammensetzung nach Anspruch 1 oder 2, dadurch **gekennzeichnet,** dass Alpha-Ketoglutarat in Form von seinem Natriumsalz zugeführt wird.

4. Verfahren zur Herstellung der Zusammensetzung nach einem der vorhergehenden Ansprüche, dadurch **gekennzeichnet,** dass Alpha-Ketoglutarat und wahlweise L-Asparagin und wahlweise Acetacetat in Wasser aufgelöst und sterilgefiltert und unmittelbar vor der therapeutischen Behandlung in kühl aufbewahrter oder gefriergetrockneter Form oder einer anderen sterilen Pulverform dem herkömmlichen Aminosäuregemisch zugesetzt werden.

5. Verwendung der Zusammensetzung nach einem der Ansprüche 1-3 zur Herstellung eines Arzneimittels zur therapeutischen, insbesondere postoperativen und posttraumatischen, parenteralen Ernährungsbehandlung.

**Patentansprüche für folgende Vertragsstaaten : ES, GR**

1. Verfahren zur Herstellung einer Zusammensetzung zur therapeutischen, insbesondere postoperativen und Posttraumatischen, parenteralen Ernährungsbehandlung, auf Basis eines herkömmlichen Aminosäuregemisches, das - ausgedrückt in g trockener Bestandteil/l wässrige Lösung - wie folgt zusammengesetzt ist:

| Glycin | 1-12 |
|---|---|
| Aspartat | 1-10 |
| Glutamat | 2-12 |
| Alanin | 2-20 |
| Arginin | 2-14 |
| Cystein/Cystin | 0,4-2,0 |
| Histidin | 2-8 |
| Isoleucin | 2-8 |
| Leucin | 2-8 |
| Lysin | 2-12 |
| Methionin | 1-6 |
| Phenylalanin | 4-10 |
| Prolin | 4-10 |
| Serin | 2-10 |
| Threonin | 2-8 |
| Tryptophan | 1-3 |
| Tyrosin | 0,2-1 |
| Valin | 2-8 |

dadurch **gekennzeichnet,** dass 5-25 g/l Alpha-Ketoglutarat und wahlweise 0,5-10 g/l L-Asparagin, 0,5-10 g/l Acetacetat, 5-30 g/l L-Glutamin oder deren Salze oder Ester in Wasser aufgelöst und sterilgefiltert und unmittelbar vor der therapeutischen Behandlung in kühl aufbewahrter oder gefriergetrockneter Form oder einer anderen sterilen Pulverform dem herkömmlichen Aminosäuregemisch zugesetzt werden.

2. Verfahren nach Anspruch 1, dadurch **gekennzeichnet,** dass 5-25, vorzugsweise 10-25 und möglichst 16,5 g/l Alpha-Ketoglutarat und wahlweise 0,5-10 g/l L-Asparagin und 0,5-10 g/l Acetacetat oder deren Salze oder Ester in Wasser aufgelöst und sterilgefiltert und unmittelbar vor der therapeutischen Behandlung in kühl aufbewahrter oder gefriergetrockneter Form oder einer anderen sterilen Pulverform dem herkömmlichen Aminosäuregemisch zugesetzt werden.

3. Verfahren nach Anspruch 1 oder 2, dadurch **gekennzeichnet,** dass Alpha-Ketoglutarat in Form von seinem Natriumsalz zugeführt wird.

4. Verwendung einer Zusammensetzung zur therapeutischen, insbesondere postoperativen und posttraumatischen, parenteralen Ernährungsbehandlung, auf Basis eines herkömmlichen Aminosäuregemisches, das - ausgedrückt in g trockener Bestandteil/l wässrige Lösung - wie folgt zusammengesetzt ist:

EP 0 318 446 B1

| | |
|---|---|
| Glycin | 1-12 |
| Aspartat | 1-10 |
| Glutamat | 2-12 |
| Alanin | 2-20 |
| Arginin | 2-14 |
| Cystein/Cystin | 0,4-2,0 |
| Histidin | 2-8 |
| Isoleucin | 2-8 |
| Leucin | 2-8 |
| Lysin | 2-12 |
| Methionin | 1-6 |
| Phenylalanin | 4-10 |
| Prolin | 4-10 |
| Serin | 2-10 |
| Threonin | 2-8 |
| Tryptophan | 1-3 |
| Tyrosin | 0,2-1 |
| Valin | 2-8 |

wobei die Zusammensetzung auch 5-25 g/l Alpha-Ketoglutarat und wahlweise 0,5-10 g/l L-Asparagin, 0,5-10 g/l Acetacetat, 5-30 g/l L-Glutamin oder deren Salze oder Ester enthält.

5. Verwendung nach Anspruch 4, wobei die Zusammensetzung 5-25, vorzugsweise 10-25 und möglichst 16,5 g/l Alpha-Ketoglutarat und wahlweise 0,5-10 g/l L-Asparagin und 0,5-10 g/l Acetacetat oder deren Salze oder Ester enthält.

6. Die Verwendung nach Anspruch 4 oder 5, wobei Alpha-Ketoglutarat in Form von seinem Natriumsalz zugeführt wird.

**Revendications**
**Revendications pour les Etats contractants suivants : AT, BE, CH, DE, FR, GB, IT, LI, LU, NL, SE**

1. Composition pour le traitement parentéral de soutien nutritif thérapeutique, en particulier postopératoire et post-traumatique, laquelle composition est à base d'un mélange classique d'amino-acides comprenant les composants suivants, exprimés en grammes de composants secs/litre de solution aqueuse :

| | |
|---|---|
| glycine | 1-12 |
| aspartate | 1-10 |
| glutamate | 2-12 |
| alanine | 2-20 |
| arginine | 2-14 |
| cystéine/cystine | 0,4-2,0 |
| histidine | 2-8 |
| isoleucine | 2-8 |
| leucine | 2-8 |
| lysine | 2-12 |
| méthionine | 1-6 |
| phénylalanine | 4-10 |
| proline | 4-10 |
| sérine | 2-10 |
| thréonine | 2-8 |
| tryptophane | 1-3 |
| tyrosine | 0,2-1 |
| valine | 2-8 |

la composition étant caractérisée en ce qu'elle contient aussi 5 à 25 g/l d'$\alpha$-cétoglutarate et facultative-

ment 0,5 à 10 g/l de L-asparagine, 0,5 à 10 g/l d'acétoacétate et 5 à 30 g/l de L-glutamine, ou leurs sels ou esters.

2. Composition selon la revendication 1, caractérisée en ce qu'elle contient 5 à 25, de préférence 10 à 25 et tout préférablement 16,5 g/l d'α-cétoglutarate et facultativement 0,5 à 10 g/l de L-asparagine et facultativement 0,5 à 10 g/l d'acétoacétate, ou de leurs sels ou esters.

3. Composition selon la revendication 1 ou 2, caractérisée en ce que l'α-cétoglutarate est apporté sous forme de son sel de sodium.

4. Procédé pour préparer une composition selon l'une quelconque des revendications précédentes, caractérisé en ce que l'on dissout de l'α-cétoglutarate et facultativement de la L-asparagine et facultativement de l'acétoacétate dans de l'eau, on stérilise par filtration et on ajoute, sous une forme stockée à froid ou lyophilisée ou sous une autre forme en poudre stérile, au mélange classique d'amino-acides, immédiatement avant le traitement thérapeutique.

5. Emploi d'une composition selon l'une quelconque des revendications 1 à 3 pour la préparation d'une préparation pharmaceutique pour le traitement parentéral de soutien nutritif thérapeutique, en particulier postopératoire et post-traumatique.

**Revendications pour les Etats contractants suivants : ES, GR**

1. Procédé pour préparer une composition pour le traitement parentéral de soutien nutritif thérapeutique, en particulier postopératoire et post-traumatique, laquelle composition est à base d'un mélange classique d'amino-acides comprenant les composants suivants, exprimés en grammes de composants secs/litre de solution aqueuse:

| | |
|---|---|
| glycine | 1-12 |
| aspartate | 1-10 |
| glutamate | 2-12 |
| alanine | 2-20 |
| arginine | 2-14 |
| cystéine/cystine | 0,4-2,0 |
| histidine | 2-8 |
| isoleucine | 2-8 |
| leucine | 2-8 |
| lysine | 2-12 |
| méthionine | 1-6 |
| phénylalanine | 4-10 |
| proline | 4-10 |
| sérine | 2-10 |
| thréonine | 2-8 |
| tryptophane | 1-3 |
| tyrosine | 0,2-1 |
| valine | 2-8 |

le procédé étant **caractérisé** en ce que l'on dissout 5 à 25 g/l d'α-cétoglutarate et facultativement 0,5 à 10 g/l de L-asparagine, 0,5 à 10 g/l d'acétoacétate, 5 à 30 g/l de L-glutamine ou leurs sels ou esters dans l'eau, on stérilise par filtration et on ajoute, sous une forme stockée à froid ou lyophilisée ou sous une autre forme en poudre stérile, au mélange classique d'amino-acides, immédiatement avant le traitement thérapeutique.

2. Procédé selon la revendication 1, **caractérisé** en ce que l'on dissout 5 à 25, de préférence 10 à 25 et tout préférablement 16,5 g/1 d'α-cétoglutarate et facultativement 0,5 à 10 g/l de L-asparagine et 0,5 à 10 g/l d'acétoacétate ou leurs sels ou esters dans l'eau, on stérilise par filtration et on ajoute, sous une forme stockée à froid ou lyophilisée ou sous une autre forme en poudre stérile, au mélange classique d'amino-acides, immédiatement avant le traitement thérapeutique.

**3.** Procédé selon la revendication 1 ou 2, **caractérisé** en ce que l'α-cétoglutarate est apporté sous forme de son sel de sodium.

**4.** Emploi d'une composition pour le traitement parentéral de soutien nutritif thérapeutique, en particulier postopératoire et post-traumatique, composition qui est à base d'un mélange classique d'amino-acides comprenant les composants suivants, exprimés en grammes de composants secs/litre de solution aqueuse:

| | |
|---|---|
| glycine | 1-12 |
| aspartate | 1-10 |
| glutamate | 2-12 |
| alanine | 2-20 |
| arginine | 2-14 |
| cystéine/cystine | 0,4-2,0 |
| histidine | 2-8 |
| isoleucine | 2-8 |
| leucine | 2-8 |
| lysine | 2-12 |
| méthionine | 1-6 |
| phénylalanine | 4-10 |
| proline | 4-10 |
| sérine | 2-10 |
| thréonine | 2-8 |
| tryptophane | 1-3 |
| tyrosine | 0,2-1 |
| valine | 2-8 |

la composition contenant également 5 à 25 g/l d'α-cétoglutarate, et facultativement 0,5 à 10 g/l de L-asparagine, 0,5 à 10 g/l d'acétoacétate, 5 à 30 g/l de L-glutamine, ou leurs sels ou esters.

**5.** Emploi selon la revendication 4, où la composition contient 5 à 25, de préférence 10 à 25 et tout préférablement 16,5 g/l de l'α-cétoglutarate, et factultativement 0,5 à 10 g/l de L-asparagine et 0,5 à 10 g/l d'acétoacétate, ou leurs sels ou esters.

**6.** Emploi selon la revendication 4 ou 5, où l'α-cétoglutarate est apporté sous forme de son sel de sodium.